# EUROPEAN PATENT APPLICATION

(11) **EP 2 574 922 A1**
(43) Date of publication of application: **03.04.2013**
(21) Application number: 11183136.8
(22) Date of filing: 28.09.2011
(51) Int. Cl.: G01N 33/44, G01N 7/14

(54) **Apparatus for determining the amount of volatile impurities in a liquid material and method for using the same**

(71) Applicant: Siemens Aktiengesellschaft, 80333 München (DE)
(72) Inventor: Grove-Nielsen, Erik, 7870 Roslev (DK); Wolf, Erik, 91341 Röttenbach (DE)

(57) **Abstract**

The invention relates to an apparatus (22) with a hermetically sealable measuring chamber (8) comprising a bottom part (5), a chamber part (6) and a measuring part (7) with a closable opening (1) for introducing a liquid material (11) into the measuring chamber (8).

The bottom part (5) is moveably arranged in the chamber part (6) and configured to reduce the pressure in the measuring chamber (8) to a predefined pressure such that the volatile impurities are released over a free surface of the liquid material as gas bubbles (12) which collapse leaving a gas volume. The measuring chamber (8) further comprises a transparent panel (9) for determining the gas volume.

Moreover, the invention relates to a method for using the apparatus (22).

## Description

The invention relates to an apparatus for determining the amount of volatile impurities in a liquid material and to a method for using the same.

It is often desirable to determine the amount of volatile impurities in a liquid material, for instance the air content in a polymer material.

A common process for manufacturing fibre reinforced composite products is the vacuum assisted resin transfer moulding (VARTM). During the VARTM process, fabrics are placed in a mould, the mould is sealed and evacuated. Then, wet resin is injected into the mould and sucked into a cavity under vacuum. Although this process is cost-saving and allows precise tolerances and detailed shaping, it can sometimes fail to fully saturate the fabrics leading to weak spots in the final shape.

Air voids or other gas voids are released in the uncured resin at low pressure which leads to lower physical strength and lower overall quality of the final component, which in turn necessitates costly repair and refinishing work.

Therefore, it is desirable to determine the amount of air voids and other gas voids in a liquid material prior to the VARTM or other production processes.

Internally, it is known to cast test panels and to evaluate the quality of the material by measuring the density of the test panels prior to a casting process of composite components. This method demands a lot of time and effort.

It is therefore an object of the present invention to provide an apparatus for determining the amount of volatile impurities in a liquid material which allows for precise measurements and is, however, compact and cost-efficient. It is a further object of the invention to provide a method for using the same which is less time consuming compared to determination methods known in the art.

The aim of the invention is achieved by the features of the independent claims.

Further aspects of the invention are subject of the dependent claims.

The apparatus for determining the amount of volatile impurities in a liquid material comprises a measuring chamber with a bottom part, a chamber part and a measuring part. The bottom part is connected to the chamber part which in turn is connected to the measuring part such that these parts enclose the measuring chamber.

Furthermore, the measuring part comprises a closable opening for introducing a liquid material having volatile impurities into the measuring chamber.

The measuring chamber further comprises one or more transparent panels for oberserving the processes going on in the measuring chamber and in particular, for measuring a gas volume resulting from gas bubbles released over a free surface of the liquid material. For this purpose a measuring scale may be arranged at the one or more transparent panels.

The measuring chamber comprises a bottom part which is moveably arranged in the chamber part and configured to reduce the pressure in the measuring chamber to a predefined pressure such that the volatile impurities are released from the liquid material as gas bubbles. By moving the moveable bottom part in such a way that the volume of the hermetically closed measuring chamber is increased, the pressure in the measuring chamber is reduced. Thus, this apparatus comprises built-in means to reduce the pressure in the measuring chamber. Therefore, it is not necessary to connect an external vacuum generator to the measuring chamber and the pressure in the measuring chamber may be reduced manually. Hence, the apparatus is effective and nevertheless cost-efficient and robust.

In an embodiment of the invention, the measuring part comprises a tubular part and the chamber part comprises a cone-shaped part. Although, these parts may have other shapes, a tubular measuring part and an at least partly cone-shaped chamber part allow for determining the amount of volatile impurities more precisely. This is because the cone-shaped form supports assembling and rising of gas bubbles released from the liquid material and the tubular form of the measuring part facilitates the measurement of a gas volume in the measuring chamber.

The chamber part may further comprise a cylindrical part and the moveable bottom part may comprise an inner piston part, an outer piston part and a control fluid having a vapor pressure, wherein the control fluid is arranged between the inner piston part and the outer piston part in the cylindrical part of the measuring chamber. These parts form a cylinder piston system, wherein the inner piston part is guided axially slidable relative to the outer piston part within the chamber part as long as the pressure in the measuring chamber is above the vapor pressure of the control fluid. That means, the control fluid is chosen such that the vapor pressure of the control fluid corresponds to the pressure intended in the measuring chamber for releasing of volatile impurities from the liquid material. Thus, a predefined constant pressure may be maintained in the measuring chamber.

A method for using the apparatus comprises the steps of filling the measuring chamber completely with a liquid material comprising volatile impurities, closing the measuring chamber hermetically sealed, reducing the pressure in the measuring chamber to a predefined pressure such that the volatile impurities are released over a free surface of the liquid material as gas bubbles, maintaining the predefined pressure in the measuring chamber for a predetermined time such that the gas bubbles collapse leaving a gas volume, and measuring the gas volume which indicates the amount of volatile impurities in the liquid material.

The measuring chamber is completely filled with the liquid material. This also ensures unchanged measurement conditions when measuring a series of liquid material samples or different liquid materials. Hence, the results of these measurements may be compared easily.

The liquid material may comprise a liquid plastic material for instance a polymeric material like a thermosetting polymer. Hence, the method may be applied in a production process of fiber reinforced composite components to evaluate the quality of a polymer melt prior to a casting process. A polymer material of a lower quality may be identified and rejected which enhances the quality of the final composite component. The overall production costs are reduced, which is especially advantageous in case of expensive and laborious processes, for instance casting processes of wind turbine rotor blades.

Furthermore, the liquid material may be heated in the measuring chamber to reduce the viscosity of the liquid material in order to facilitate releasing of gas bubbles.

In the following, the invention will be described by way of example in more detail.

The drawings show preferred configurations and do not limit the scope of the invention. The same reference numerals are used in the drawings for elements having the same function.
**FIG 1** shows schematically an apparatus according to an embodiment of the invention
**FIG 2a, 2b, 2c** show schematically the method using the apparatus shown in FIG 1

Although the apparatus and method herein explained may be used to determine the amount of volatile impurities of various liquid materials, they will be described in the following using a liquid polymer as example of such a liquid material.

Volatile impurities in a liquid polymer comprise gaseous components, for instance trapped air. Furthermore, the volatile impurities may comprise liquid components which volatilize, if the material is exposed to a vacuum, i.e. to a pressure which is below the atmospheric pressure of 1000 mbar. This happens for instance during a casting process of a composite component. An example of such a liquid component in this context is a reactive diluent in an epoxide resin.

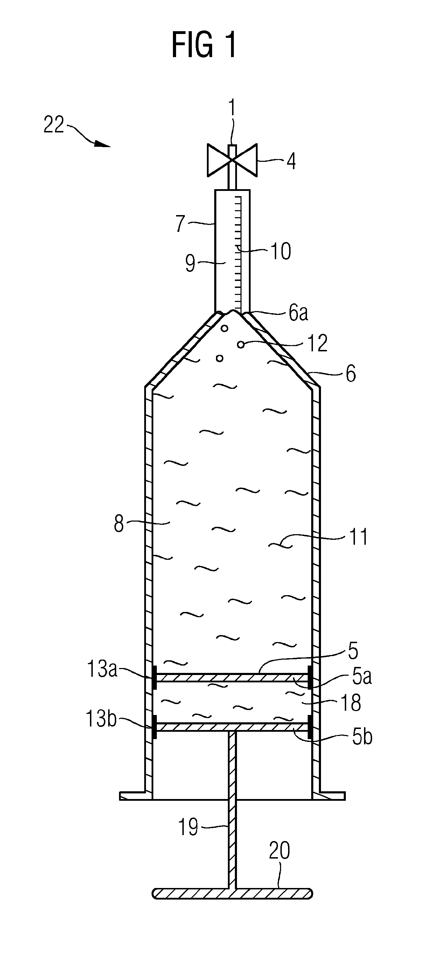

In the following, an apparatus 22 for determining the amount of volatile impurities in a liquid material according to an embodiment of the invention will be described in more detail, referring to **FIG 1****.**

The apparatus 22 is preferably portable and comprises a hermetically sealable measuring chamber 8 with a bottom part 5, a chamber part 6 and a measuring part 7, wherein the bottom part 5 is connected to the chamber part 6 and the chamber part 6 is connected to the measuring part 7.

In the embodiment, shown in FIG 1, the chamber part 6 comprises a cylindrical part and cone-shaped part. A tapered end 6a of the cone-shaped part 6 is connected to an end 7a of the measuring part 7, wherein the measuring part 7 is tubular. The opposite end of the cone-shaped part is connected to the cylindrical part.

An inlet opening 1 is arranged in the tubular measuring part 7, preferably, at a free end of this tubular measuring part 7. The inlet opening 1 is used to introduce a liquid material 11 into the measuring chamber 8. Besides, the inlet opening 1 is used to discharge the liquid polymer 11 from the measuring chamber 8 after having measured the amount of volatile impurities. The inlet port 1 comprises a valve 4, for instance a magnetic valve or other means suitable for closing the measuring chamber 8 hermetically.

Moreover, the measuring chamber 8 comprises a bottom part 5 which is moveably arranged in the chamber part 6. The moveable bottom part 5 is configured to reduce the pressure in the measuring chamber 8 by being moved in such a way that the volume of the measuring chamber 8 increases. In other words, the pressure in the measuring chamber 8 may be reduced by moving the moveable bottom part 5 in a direction away from the tubular measuring part 7.

The moveable bottom part 5 comprises an inner piston part 5a, an outer piston part 5b and a control fluid 18 having a defined gas pressure. This control fluid 18 is arranged between the inner piston part 5a and the outer piston part 5b. A first sealing 13a is arranged between the inner piston part 5a and the chamber part 6 and a second sealing 13b is arranged between the outer piston part 5b and the chamber part 6.

The inner piston part 5a is guided within the chamber part 6 in an axially slidable manner relative to the outer piston part 5b. In a fluidic state, the control fluid stiffly connects the inner piston part 5a and the outer piston part 5b. Thus, by moving the outer piston part 5b, the complete moveably bottom part 5a, 18, 5b, 5c is moved and a liquid polymer material 11 may be sucked into the measuring chamber 8 through the inlet opening 1. By further moving the outer piston part 5b, the pressure in the measuring chamber 8 is reduced.

In order to allow for reducing the pressure in the measuring chamber 8 to the intended predefined pressure, the control fluid 18 has a vapor pressure which corresponds to the intended predefined pressure. Vapor pressure means a substance and temperature depending gas pressure and denotes the ambient pressure, below which a fluid starts vaporizing at a constant temperature.

Thus, the control fluid 18 starts boiling, if the intended predefined pressure is reached in the measuring chamber 8. If the outer piston 5b is further moved away from the inlet opening 1, the inner piston part 5a will no longer follow and the pressure in the measuring chamber 8 will not be reduced any further. As a result, a predefined constant pressure is generated within the measuring chamber 8.

Optionally, a rod 19 and/or a handle 20 may be connected to the moveable bottom part 5 to faciltate moving of the bottom part 5.

Moreover, a transparent panel 9 is arranged in the tubular measuring part 7 for observing at least a part of the measuring chamber 8.

Besides, a further transparent panel may be arranged in the chamber part 6 for monitoring the processes going on in the measuring chamber 8. The transparent panel 9 and/or the further transparent panel may be configured as a window of any shape and may include any transparent material, for instance glass or acryl glass. Alternatively, the tubular measuring part 7 and/or the chamber part 6 may be made of a transparent material for observing the inside of the measuring chamber 8.

In order to facilitate the measurement, a measuring scale 10 is arranged at the transparent panel 9.

Preferably, the measuring chamber 8 comprises a heating device (not shown) for heating the liquid polymer 11 in the measuring chamber 8 to reduce the viscousity thereof. The heating device may, for instance, comprise a battery-powered heating coil connected to the chamber part 6.

The measuring chamber 8 may be made, at least partly, of a metallic material, for instance aluminium, high-grade steel or copper, in order to facilitate the heat transfer from the heating device to the liquid polymer 11 in the measuring chamber 8.

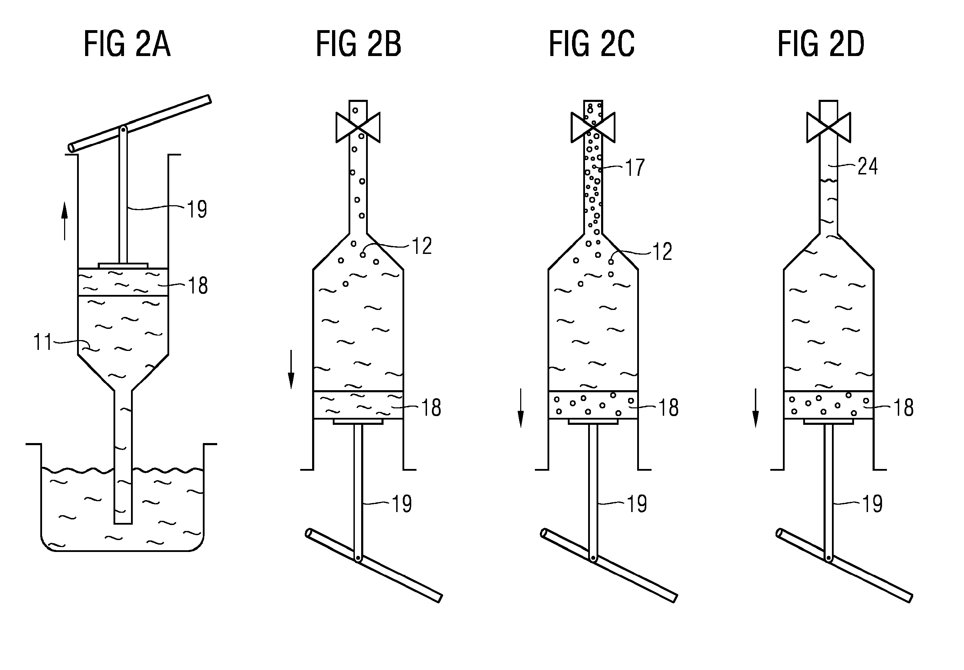

In the following, it will be described how the aforementioned method may be carried out using the apparatus 22, shown in FIG 1. Reference is made to the drawings, especially to FIG 2a, FIG 2b and FIG 2c.

As indicated by an arrow in **FIG 2a****,** the moveable bottom part 5 is moved to suck the liquid material from a container into the measuring chamber 8. The liquid polymer material, for instance an epoxide resin, is introduced through the inlet opening 1.

Then, the apparatus 22 is turned such that the tubular measuring part 7 faces upwards. Air which has been kept in the measuring chamber 8 is discharged or pressed out through the inlet opening 1. Thus, the measuring chamber is filled completely.

As schematically shown in **FIG 2b**, the measuring chamber is then closed hermetically using a valve 4 connected to the inlet opening 1, wherein "hermetically" or "hermetically sealed" means leak-proof and vacuum tight.

After having closed the measuring chamber, the moveable bottom part 5 is moved by pulling the rod 19 downwards, as indicated by an arrow in FIG 2b. That means the rod 19 and thus, the moveable bottom part 5, is pulled in a direction away from the inlet opening 1. Thereby, the pressure in the measuring chamber 8 is reduced to a predefined pressure such that the volatile impurities are released from the liquid polymer 11 as gas bubbles 12.

As schematically shown in **FIG 2c****,** the control fluid 18 starts boiling at the predefined pressure intended in the measuring chamber 8 for the bubble 12 formation and for the measurement of the remaining gas volume in the measuring chamber 8. If this pressure is reached, the inner piston part 5a does not move any further and the pressure in the measuring chamber 8 remains constant. Gas bubbles 12 in the liquid material expand, migrate into other gas bubbles, assemble and rise upwardly. The gas bubbles 12 are released over a free surface of the liquid polymer 11 in the measuring chamber and a gas bubble column 17 builds-up in the tubular measuring part 7.

The predefined pressure is maintained for a predetermined time. Finally, the gas bubbles collapse.

As shown in **FIG 2d****,** the gas bubble column 17 disappears leaving a certain gas volume in the tubular measuring part 7. The gas volume is observable as a "free" or transparent gas column 24. This involves a remaining filling level of the measuring chamber 8 which is reduced compared to the filling level at the beginning of the measuring procedure. This remaining filling level or the height of the transparent gas column 24 may be read off by means of the transparent panel 9 having a measuring scale 10 printed thereon.

The gas volume is measured after a predetermined time which may also be referred to as waiting time. The predetermined time depends on the liquid material 11 to be evaluated and on of the temperature of the liquid material 11. By way of example, in case of a liquid polymer at a temperature of 40 to 60 degrees Celsius a waiting time of a few minutes may be sufficient, wherein at a temperature of about 20 degrees Celsius a waiting time of about one hour could be required.

The disclosure is illustrative only and changes may be made in detail, especially in matters of shape, size and arrangement of parts within the principles of the invention in the full extent indicated by the meaning in which the appended claims are expressed.

## Claims

1. Apparatus (22) for determining the amount of volatile impurities in a liquid material with
- a measuring chamber (8) comprising a bottom part (5), a chamber part (6) and a measuring part (7),
- wherein the bottom part (5) is connected to the chamber part (6) and the chamber part (6) is connected to the measuring part (7) such that the parts (5, 6, 7) enclose the measuring chamber (8) hermetically sealable,
- wherein the measuring part (7) comprises a closable opening (1) for introducing a liquid material (11) comprising volatile impurities into the measuring chamber (8),
- wherein the bottom part (5) is moveably arranged in the chamber part (6) and configured to reduce the pressure in the measuring chamber (8) to a predefined pressure such that the volatile impurities are released over a free surface of the liquid material as gas bubbles (12) which collapse leaving a gas volume, and
- wherein the measuring chamber (8) further comprises a transparent panel (9) for determining the gas volume which indicates the amount of volatile impurities in the liquid material 11.

2. Apparatus (22) according to claim 1, **characterized in that** the chamber part (6) comprises a cylindrical part and a cone-shaped part, wherein the cylindrical part is connected to an end of the cone-shaped part, the cone-shaped part having a further end (6a) which is tapered, and
wherein the measuring part (7) is a tubular measuring part, an end of which is connected to the tapered end (6a) of the cone-shaped part (6).

3. Apparatus (22) according to claim 1 or claim 2, **characterized in that**
the moveable bottom part (5) comprises an inner piston part (5a), an outer piston part (5b), and a control fluid (18) arranged between the inner piston part (5a) and the outer piston part (5b),
- wherein the control fluid (18) is chosen such that the vapor pressure thereof corresponds to the predefined pressure intended in the measuring chamber for the liquid material releasing the volatile impurities.

4. Apparatus (22) according to one of the preceding claims, **characterized in that**
the transparent panel (9) comprises a measuring scale (10) for measuring the gas volume.

5. Apparatus (22) according to one of the preceding claims, **characterized in that**
the closable opening (1) comprises a valve (4).

6. Apparatus (22) according to one of the preceding claims, **characterized in that**
the measuring chamber (8) comprises a metallic material.

7. Apparatus (22) according to one of the preceding claims, further comprising a heating device for heating the measuring chamber (8) to reduce the viscosity of the liquid material.

8. Method for determining the amount of volatile impurities in a liquid material using the apparatus (22) according to one of the preceding claims comprising the steps of:
- filling the measuring chamber completely with a liquid material comprising volatile impurities,
- closing the measuring chamber hermetically sealed,
- reducing the pressure in the measuring chamber to a predefined pressure such that the volatile impurities are released over a free surface of the liquid material as gas bubbles,
- maintaining the predefined pressure for a predetermined time such that the gas bubbles collapse leaving a gas volume, and
- measuring (130) the gas volume which indicates the amount of volatile impurities in the liquid material.

9. Method according to claim 8, **characterized in that** the step of filling the measuring chamber completely with a liquid material having volatile impurities comprises sucking the liquid material into the measuring chamber.

10. Method according one of claims 8 to 9,
further comprising the step of positioning the apparatus such that the measuring part (7) faces upwards and the moveably bottom part (5) faces downwards.

11. Method according one of claims 8 to 10, **characterized in that** the liquid material comprises a plastic material.

12. Method according one of claims 8 to 11, **characterized in that** the liquid material is heated in the measuring chamber to reduce the viscosity of the liquid material.
